Europäisches Patentamt

European Patent Office

Office européen des brevets

(11) Veröffentlichungsnummer: **0 382 905**
**A2**

## (12) EUROPÄISCHE PATENTANMELDUNG

(21) Anmeldenummer: **89121659.0**

(22) Anmeldetag: **23.11.89**

(51) Int. Cl.5: **G01N 1/10, G01N 1/34**

(30) Priorität: **17.02.89 DE 3904872**

(43) Veröffentlichungstag der Anmeldung:
**22.08.90 Patentblatt 90/34**

(84) Benannte Vertragsstaaten:
**AT BE CH DE ES FR GB IT LI LU NL SE**

(71) Anmelder: **REMEDIA AG**
**Waldenburgerstrasse 1**
**CH-4006 Basel(CH)**

(72) Erfinder: **Schlüter, Gert**
**Im Höfle 22**
**D-7802 Gundelfingen(DE)**

(74) Vertreter: **von Hellfeld, Axel, Dr. Dipl.-Phys., et al**
**WUESTHOFF & WUESTHOFF**
**Schweigerstrasse 2**
**D-8000 München 90(DE)**

(54) **Verfahren und Vorrichtung zum Sammeln und Abtrennen von Partikeln aus Flüssigkeit für die medizinische Diagnose.**

(57) Bei einem Verfahren zum Sammeln und Abtrennen von Zellen, Partikeln od. dgl. aus Körperflüssigkeit od. dgl. für die mikroskopische Diagnostik wird aus einer Zellflüssigkeit, z.B. Urin, Flüssigkeit durch Kapillarwirkung durch eine flüssigkeitsdurchlässige, das zu untersuchende Zellmaterial zurückhaltende Oberfläche (17) hindurchgesaugt. Eine dazu geeignete Vorrichtung weist einen mit einer oberen und einer unteren Öffnung versehenen Filterbehälter (2) auf, dessen untere Öffnung von einem flüssigkeitsdurchlässigen Zellträgerelement (10) abgedeckt ist, wobei im Filterbehälter (2) mindestens ein Filterabschnitt (11) und eine Saugeinlage (12) angeordnet sind.

FIG.1

## Verfahren und Vorrichtung zum Sammeln und Abtrennen von Partikeln aus Flüssigkeit für die medizinische Diagnose

Die Erfindung bezieht sich auf ein Verfahren zum Sammeln und Abtrennen von Partikeln od. dgl. aus Flüssigkeit für die medizinische Diagnose, insbesondere die mikroskopische Diagnostik.

Bekannte Zellabscheidungsverfahren aus Körperflüssigkeiten arbeiten in der Regel mittels Filtration, Zentrifugalkraft oder Sedimentation. Hierfür sind eine Reihe von Verfahren und Geräten entwickelt worden. Der Mehrzahl dieser Verfahren liegt die Aufgabe zugrunde, Flüssigkeiten von Partikeln zu säubern (Wasser aufbereiten) und die Flüssigkeit dann zu beseitigen. Hierbei wird die gereinigte Flüssigkeit weiterverwendet und die Partikel werden verworfen. Bei solchen Verfahren ist eine Partikelgewinnung aus den verwendeten Filtern, z.B. Mehrschicht-oder Tiefenfilter, auch nicht möglich.

In der Labormedizin arbeitet man bei der Untersuchung von Körperflüssigkeiten mit sehr unterschiedlichen Methoden, die jeweils von der diagnostischen Aufgabe bzw. vom Krankheitsbild bestimmt werden. Es ist bekannt, Flüssigkeitsproben für die Gewinnung von Zellen zu zentrifugieren. Das Sediment wird dann über Objektträgerausstriche ausgewertet. Gemäß dem Stand der Technik wird vorzugsweise eine spezielle Art der Zentrifugation angewendet, bei der das Zellmaterial aus einer eingegebenen Probe direkt auf einem Objektträger deponiert wird. Die (Körper-) Flüssigkeit wird bei diesem Prozeß über den Zentrifugationsvorgang von den Partikeln getrennt und in einem Vliespapier gesammelt, welches zusammen mit dem Objektträger in der Zentrifuge eingespannt ist.

Neben den Zentrifugationsmethoden werden außerdem für die Zellgewinnung Filtrationsmethoden angewendet, die sich überwiegend der Membranfiltertechnik bedienen.

Sollen aus Körperflüssigkeiten, z.B. aus Urin, pathologische Prozesse, wie z.B. Neoplasien, nachgewiesen werden, müssen die für die zytologische Diagnose relevanten Zellen in hoher Anzahl und ohne Selektion aus der Gesamtprobe isoliert werden. Der Zytologe stellt hierbei an das Untersuchungsmaterial und an die Zellgewinnungsmethode besonders hohe Anforderungen. Dabei ist sehr wichtig, daß nach dem Gewinnen der Zellen deren Ausgangszustand voll erhalten bleibt, d.h. daß die Feinmorphologie der Zellen dem Zustand entspricht, den diese schon bei der Abschilferung vom Gewebe in die Flüssigkeit hinein hatten. Bei Körperflüssigkeitsproben handelt es sich in der Regel um sehr unterschiedlich zusammengesetztes Material. Ascites ist häufig zell-, blut- und proteinreich und hat eine hohe Viskosität. Frauenurine sind zellreicher und viskoser als Männerurine. Entzündliche Prozesse kontaminieren die Proben mit Erythrozyten und Leukozyten und beeinflussen auch die Fließgeschwindigkeit. Genauso können Proben mit Protein-, Fibrin- und Blutkoagular durchsetzt sein. Bakterien zerstören Zellen und führen zu einem Durchsetzen der Proben mit Detritus.

Für die Zytologie von Körperflüssigkeiten, und das gilt neben Urin für alle, auch für Liquor, für Ergüsse, für Ascites und für Bauchhöhlenlavage, muß - unabhängig von der Beschaffenheit der Flüssigkeit - ein gut erhaltenes Zellmaterial gewonnen werden können.

Vorbekannte Verfahren, die für die Partikel- und/oder Zellgewinnung eingesetzt werden, sind noch mit Nachteilen belastet. So ist bekannt, daß bei Zentrifugationsvorgängen an den Wandungen der Zentrifugengläser, abhängig vom Adhäsionsverhalten der Zellen, vergleichsweise viel Zellmaterial verlorengehen kann.

Bekannte Membranfilter weisen ebenfalls erhebliche Nachteile auf; insbesondere sind sie bei sehr zellreichen Proben oder bei hochviskosen Lösungen nicht einsetzbar. Empfindliche oder vorgeschädigte Zellen werden bei beiden Methoden in hohem Anteil zerstört oder verändert. Nachteilig sind auch Filtrationsverfahren und -Vorrichtungen, bei denen Zellen verlorengehen. Dadurch wird nämlich das Gesamtbild des zytologischen Präparates verfälscht und es besteht die Gefahr, daß kleinzellige Karzinome negativ selektiert werden.

Bekannte medizinische Diagnosemethoden stellen an ein Zellgewinnungsverfahren bzw. die dazu gehörenden Vorrichtungen folgende Anforderungen, die aber in aller Regel nicht oder nur unvollständig erfüllt werden können:
- Abtrennung aller für die Diagnostik wichtigen Zellen, ohne Zell-Selektion oder Zell-Verlust. - Keine Einschränkung hinsichtlich der Partikelgröße. Zellen mit Durchmessern von 7 $\mu$m und weniger müssen im Anteil genauso erfaßt werden wie Zell-Konglomerate über 100 $\mu$m Durchmesser.
- Die Viskosität einer Lösung (aus Körperflüssigkeit) darf keinen Einfluß auf die Gewinnungsrate der Zellen haben.
- Das Verfahren muß schnell, effektiv und reproduzierbar arbeiten und auch für Reihenuntersuchungen geeignet sein.
- Zum Schutz des Anwenders muß gesichert sein, daß Berührungskontakte mit der zu untersuchenden Flüssigkeit durch eine die Untersuchung ausführende Person weitestgehend ausgeschlossen ist (Infektionsprophylaxe).
- Die Flüssigkeitsprobe muß nach Abtrennen der Partikel sicher entsorgt oder aber auch für andere Untersuchungen in einem geschlossenen System sicher verwahrt werden können.
- Schließlich sollen von der Flüssigkeit abgetrennte Partikel (Zellen) direkt nach der Gewinnung in einer geeigneten Flüssigkeit, z.B. Alkohol, fixiert bzw. konserviert werden können.

Der Erfindung liegt die Aufgabe zugrunde, ein Verfahren und eine Vorrichtung zum Sammeln und Abtrennen von Partikeln aus einer Flüssigkeit für die medizinische Diagnose zu schaffen, welches bzw. welche in einfacher und kostengünstiger Weise zuverlässige medizinische Diagnosen ermöglicht.

Das erfindungsgemäße Verfahren zur Lösung dieser Aufgabe ist dadurch gekennzeichnet, daß die Flüssigkeit mittels eines saugfähigen Materials durch eine Oberfläche gesaugt wird, an der die Partikel haften bleiben.

Eine erste erfindungsgemäße Vorrichtung zur Lösung dieser Aufgabe zeichnet sich aus durch
- einen ersten Behälter, in dem eine die Flüssigkeit ansaugende Saugeinlage angeordnet ist, und
- eine im Saugweg der Flüssigkeit angeordnete Oberfläche, die für die Flüssigkeit, nicht aber für die Partikel, durchlässig ist.

Eine zweite erfindungsgemäße Vorrichtung zur Lösung der oben gestellten Aufgabe zeichnet sich aus durch
- einen Behälter, der in der Lage ist, selbsttätig sein Volumen zu vergrößern, um die Flüssigkeit anzusaugen, und
- eine im Saugweg der Flüssigkeit angeordnete Oberfläche, die für die Flüssigkeit, nicht aber für die Partikel, durchlässig ist.

In einer bevorzugten Ausgestaltung dieser Vorrichtung ist vorgesehen, daß der Behälter sein Volumen unter Wirkung einer Feder oder aufgrund seiner Eigenelastizität vergrößert.

Das erfindungsgemäße Verfahren und die Vorrichtung zur Durchführung eines solchen Verfahrens weisen eine Reihe von Vorteilen auf. Es können präzise auswertbare Proben gewonnen werden, ohne daß aufwendige und kostenintensive Vorrichtungen erforderlich sind. Das Verfahren kann einfach und in kurzer Zeit durchgeführt werden, ohne besonders geschultes Personal zur erfordern. Weiterhin ist das erfindungsgemäße Verfahren bzw. die Vorrichtung bezüglich Partikel (Zellen) unterschiedlicher Größe und Art relativ wenig selektiv, d.h. die gewonnene Partikelverteilung spiegelt die tatsächlichen Verhältnisse in der zu untersuchenden Flüssigkeitsprobe weitestgehend wieder.

Auch ist das erfindungsgemäße Verfahren relativ schonend, d.h. das zu untersuchende Zellmaterial bleibt weitestgehend unzerstört.

Auch ermöglicht die Erfindung eine verhältnismäßig einfache Anpassung an verschiedene Diagnosemethoden, z.B. an die Zellgewinnung aus dem Urogenitaltrakt.

Der hier verwendete Begriff "Flüssigkeit" ist umfassend zu verstehen. Er umfaßt jede Art von Körperflüssigkeit, d.h. vom menschlichen oder tierischen Körper erzeugte Flüssigkeit, als auch Verarbeitungsergebnisse solcher Flüssigkeiten, z.B. Lösungen von Körperflüssigkeiten.

Der Begriff "Partikel" im Sinne der Erfindung ist umfassend zu verstehen und meint insbesondere, aber nicht nur, Zellen.

In einer besonders bevorzugten Ausgestaltung des erfindungsgemäßen Verfahrens bzw. einer Vorrichtung zur Durchführung eines solchen Verfahrens ist vorgesehen, daß die Flüssigkeit mittels Kapillarwirkung durch die Oberfläche gesaugt wird, an der die Partikel (insbesondere Zellen) haften bleiben.

Statt durch Kapillarwirkung kann die Saugfähigkeit des saugfähigen Materials auch anders erzeugt werden, zum Beispiel durch eine chemische Umsetzung (hydrophiles Material) oder durch einen selbsttätigen Expansionsvorgang, bei dem auf einer Seite der Oberfläche ein Unterdruck erzeugt wird.

Bevorzugt ist die Oberfläche nicht integral mit dem saugfähigen Material ausgebildet, sondern als eine getrennte Schicht. Als besonders geeignet haben sich Kunststoff- und Acetatgewebe erwiesen. Eine besonders zuverlässige Messung ermöglicht ein Acetatgewebe.

Die Erfindung ermöglicht es, mit geringem Aufwand Zellmaterial auf eine Oberfläche (Bereitstellungsfläche) aufzubringen, bedarfsweise zu fixieren und anschließend auf einen Objektträger aufzustreichen.

Wie Versuche gezeigt haben, erfolgt eine Abtrennung aller für die Diagnostik wichtigen Zellen ohne praktisch ins Gewicht fallenden Verlust an Zellmaterial. Eine vorteilhafte Weiterbildung des Verfahrens besteht darin, daß vor dem Abstreifen des zu untersuchenden Zellmaterials auf einen Objektträger wesentliche Teile der bei der Probenahme mit angesaugten Flüssigkeit entfernt werden. Das begünstigt nicht nur ein sauberes Arbeiten mit entsprechend geringer Infektionsgefahr für die Bedienungsperson, sondern ermöglicht auch, das gewonnene Zellmaterial, z.B. per Post, zu versenden.

Eine vorteilhafte Weiterbildung des erfindungsgemäßen Verfahrens besteht darin, daß das Ansaugen des zu untersuchenden Zellmaterials mit Hilfe eines erheblichen Volumenvergrößerns eines Filterabschnittes verstärkt wird. Durch dieses besondere Vergrößern des Volumens des Filterabschnittes wird die Saug wirkung bei der Beschickung der Bereitstellungsfläche so stark vergrößert, daß ein gutes Abtrennen aller für die Diagnostik wichtigen Zellen bei schneller und effektiver Arbeitsweise des Verfahrens stark begünstigt wird.

Nach einer zusätzlichen Weiterbildung des Verfahrens wird das auf der Oberfläche gesammelte, zu untersuchende Zellmaterial dadurch transportfähig gemacht, daß es, gegebenenfalls nach Fixierung, in einen Verschlußbehälter eingeführt wird, z.B. nachdem die überflüssige Flüssigkeit entfernt worden ist. Das zu untersuchende Zellmaterial befindet sich dann, gegen äußere Einflüsse geschützt, in einem geschlossenen, transportfähigen Behälter.

Es kann erwünscht sein, daß zusätzlich zu dem auf der Bereitstellungsfläche bereits befindlichen, zu untersuchenden Zellmaterial weitere Untersuchungsflüssigkeit bei der Probe behalten bzw. mittransportiert werden soll. In einem solchen Fall ist es vorteilhaft, daß innerhalb eines die Bereitschaftsfläche aufnehmenden Behälters, vorzugsweise des Verschlußbehälters, zu untersuchende Flüssigkeit zusätzlich zugegeben wird.

Eine bevorzugte Ausgestalung der erfindungsgemäßen Vorrichtung für eine solche Vorrichtung besteht darin, daß die Vorrichtung einen mit einer oberen und unteren Öffnung versehenen Filterbehälter aufweist, dessen untere Öffnung von einem flüssigkeitsdurchlässigen, aber zellenundurchlässigen Zellträgerelement abgedeckt ist, wobei im Filterbehälter - hintereinander von unten nach oben - hinter dem Zellträgerelement mindestens einen Filterabschnitt und danach eine Saugeinlage angeordnet sind. Auf dem Zellträgerelement ist die oben genannte Oberfläche ausgebildet.

Wenn man das untere, mit dem Zellträgerelement versehene Ende dieser Vorrichtung in einen z.B. mit Urin gefüllten Behälter eintaucht, wird ein entsprechender Flüssigkeitsanteil durch das Trägerelement und durch einen Filterabschnitt hindurch in die Saugeinlage hineingesaugt, ohne daß es irgendwelcher zusätzlicher Hilfsmaßnahmen bedarf. Die aus der Flüssigkeit abzutrennenden Zellen, Partikel od. dgl. zu untersuchendes Zellmaterial legen sich am Zellträgerelement an, wobei der Sammel- und Abtrennvorgang schonend und dennoch zügig und schnell erfolgt.

Insbesondere für das Versenden von abgetrenntem Zellmaterial ist es vorteilhaft, wenn die Vorrichtung einen Verschlußbehälter für das untere, das Zellträgerelement aufweisende Ende des Filterbehälters hat.

Insbesondere bei der letztgenannten Ausbildung der Vorrichtung ist es vorteilhaft, das zwischen dem Verschluß- und dem Filterbehälter eine Flüssigkeitsdichtung vorgesehen ist, vorzugsweise dadurch, daß wenigstens beim unteren Ende des Filterbehälters eine Flüssigkeitsdichtung angeordnet ist. Wenn der Verschlußbehälter, wie zum Transport üblich, an seinem dem Zellträgerelement gegenüberliegenden Ende verschlossen und mit seinem anderen Endbereich oder seinem Mittelbereich flüssigkeitsdicht mit dem Filterbehälter in Verbindung steht, ergibt sich ein das Zellträgerelement abdichtend umschließendes Gehäuse. Dieses schützt das zu untersuchende Zellmaterial vor unerwünschten Einflüssen.

Eine vorteilhafte Weiterbildung der erfindungsgemäßen Vorrichtung besteht darin, daß der Filterbehälter an seinem oberen, das heißt dem Zellträgerelement abgewandten Ende einen gelochten Deckel od. dgl. oberen Anschluß hat und diese Lochung vorzugsweise als Einsteck- oder Entnahmeöffnung für die Saugeinlage ausgebildet ist. Dies erlaubt ein leichtes Entfernen dieser Saugeinlage und damit die Entfernung eines ganz erheblichen Teils der Körperflüssigkeit od. dgl. Flüssigkeit, aus der das Zellmaterial abgetrennt wurde.

Eine besonders vorteilhafte Weiterbildung der Vorrichtung besteht darin, daß zumindest ein dem Zellträger naher Filterab schnitt aus sich bei Kontakt mit Flüssigkeit schwammartig entfaltendem Werkstoff, vorzugsweise aus regenerierter, verpreßter Zellulose besteht. Wenn ein solcher Filterabschnitt sich beim Kontakt mit Flüssigkeit schwammartig in seinem Volumen vergrößert, wird die Saugwirkung der Vorrichtung verstärkt. Als Werkstoff für ein schwammartig sich entfaltender Filterabschnitt hat sich regenerierte, verpreßte Zellulose als besonders geeignet herausgestellt. Sie besitzt auch so viel Eigenstabilität, daß sie das aus Gewebe, insbesondere dichtem Acetatgewebe bestehende Zellträgerelement im Inneren des Filterbehälters ausreichend festklemmen und wandbündig verdichten kann. Dies trägt mit dazu bei, daß der Aufbau der gesamten Vorrichtung nicht nur sehr einfach ist, sondern auch unerwünschte zusätzliche Halterungen für dieses Acetatgewebe od. dgl. die das Verfahren zum Sammeln und Abtrennen von Zellen beeinträchtigen können, nicht benötigt werden.

Nachstehend wird die Erfindung anhand von Ausführungsbeispielen in Verbindung mit der Zeichnung noch näher beschrieben. In unterschiedlichen Maßstäben und schematisiert zeigen:

Fig. 1 einen Längsschnitt durch eine Vorrichtung zum Sammeln von Zellen od. dgl., auf deren unteres Ende ein Verschlußbehälter aufgesteckt ist,

Fig. 2 den Verschlußbehälter nach Fig. 1,

Fig. 3 den leeren Filterbehälter gemäß Fig. 1,

Fig. 4 den in den leeren Filterbehälter nach Fig. 3 einzubringenden Filterbehälter-Inhalt, jedoch ohne ein Zellträgerelement,

Fig. 4a die auf eine Saugeinlage gemäß Fig. 4 aufzusetzende Muffe,

Fig. 5 einen Verschlußstab, der in den Filterbehälter nach dem Entfernen seiner Saugeinlage einzuführen ist,

Fig. 6 den Inhalt des Filterbehälters, nämlich eine Saugeinlage, ein Filterabschnitt sowie ein Zellträgerelement in auseinandergezogener Darstellung,

Fig. 7 den unteren Teil des Filterbehälter-Inhalts ähnlich Fig. 6, ebenfalls in auseinandergezogener Darstellung, und

Fig. 8 ein weiteres Ausführungsbeispiel einer erfindungsgemäßen Vorrichtung.

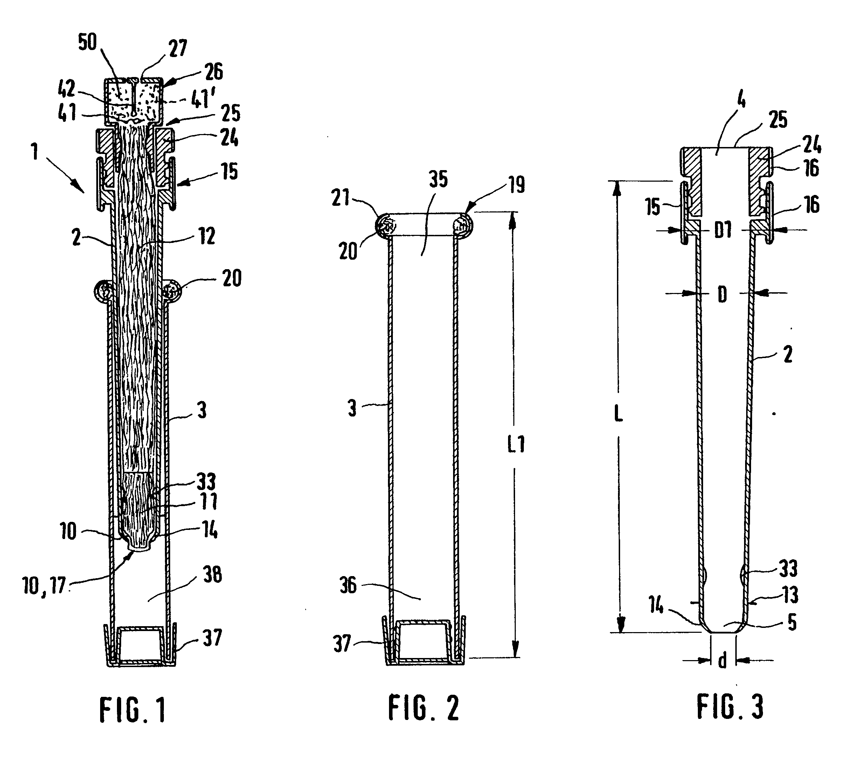

Eine im ganzen mit 1 bezeichnete Vorrichtung weist einen Filterbehälter 2 sowie einen Verschlußbehälter 3 auf (Fig. 1 und 2). Diese Vorrichtung 1 dient zum Sammeln und Abtrennen von Zellen und Partikeln aus Körperflüssigkeiten od. dgl. für die mikroskopische Diagnostik. Der Filterbehälter 2 hat eine obere und eine untere Öffnung 4 und 5. Er ist im wesentlichen rohrförmig ausgebildet (Fig. 3). Wie gut aus Fig. 1, 6 und 7 erkennbar ist, ist die untere Öffnung 5 von einem Zellträgerelement 10 aus dichtem Acetatgewebe abgedeckt. Im Filterbehälter 2 befinden sich, von unten nach oben hintereinander angeordnet, ein Filterabschnitt 11 und, daran anschließend, eine Saugeinlage 12.

Der (leere) Filterbehälter 2 ist im Ausführungsbeispiel nach Fig. 3 gegenüber einer möglichen, praktischen Ausführung nur wenig verkleinert dargestellt. Er ist, wie nachstehend noch näher erläutert, als Handgerät mit entsprechenden Abmessungen und Griffhilfen ausgestattet. Dabei besteht der Filterbehälter 2 vorzugsweise aus Polyethylen und seine Gesamtlänge L beträgt z.B. 110 mm und sein innerer Durchmesser D z.B. 10,5 mm. Der Filterbehälter 2 hat eine ringförmige Flüssigkeitsdichtung 13, die nahe dem des unteren Ende 5 angebracht ist. Das untere Ende 5 des Filterbehälters 2 weist eine konische Verengung 14 auf, so daß sich am unteren Ende 5 des Filterbehälters 2 eine lichte Öffnung 5 mit einem Durchmesser d von z.B. 6,5 mm ergibt. Beim oberen Ende 4 ist der Filterbehälter 2 über die axiale Länge eines Griffabschnittes 15 mit einem vergrößerten Außendurchmesser D 1 versehen. Dort sind radiale Rippen 16 oder eine entsprechende Riffelung vorgesehen. Sowohl die Durchmesservergrößerung als auch die Rippen 16 sollen die Handhabung vereinfachen.

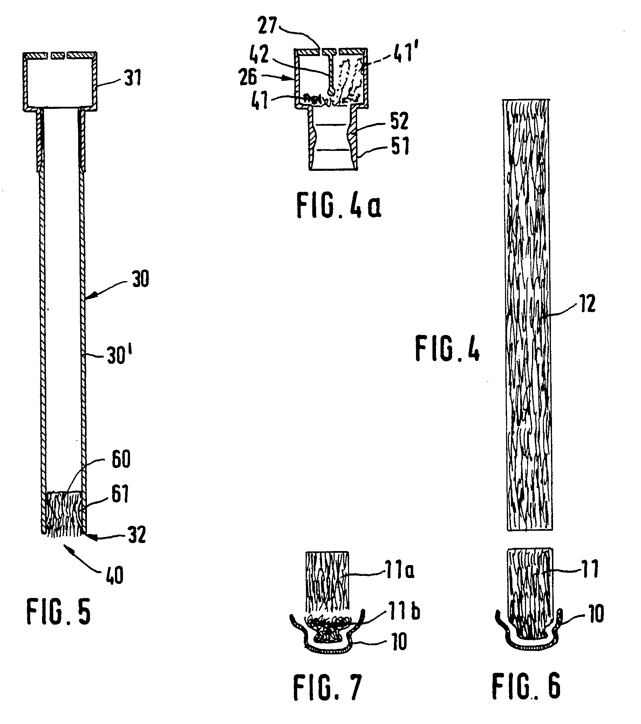

In der konischen Verengung 14 des Filterbehälters 2 ist, wie aus Fig. 1 und vergrößert gut aus Fig. 6 und 7 erkennbar ist, ein Filterabschnitt 11 untergebracht, der z.B. etwa 20 mm Länge hat. Wie gut aus Fig. 6 erkennbar, besteht der Filterabschnitt 11 aus faserigem Werkstoff mit in Längsrichtung ausgerichteten Fasern hochsaugfähigem Werkstoff. Die im Filterbehälter 2 untergebrachten Teile, also das aus einem Abschnitt von dichtem Acetatgewebe bestehende Zellträgerelement 10, der Filterabschnitt 11 und die Saugeinlage 12 sind innerhalb des Filterbehälters 2 lagefixiert.

Mit dem vorbeschriebenen Filterbehälter 2 läßt sich bereits ein Verfahren zum Sammeln und Abtrennen von Zellen und Partikeln aus Flüssigkeit für mikroskopische Diagnostik ausführen, wenn z.B. zwischen dem Sammeln und Abtrennen der Zellen aus der Flüssigkeit und dem Aufstreichen der gesammelten Zellen und Partikel auf einen Objektträger keine fallenden Zeiträume und/oder keine Transportwege überbrückt werden müssen. Man kann dann z.B. den Filterbehälter 2 in ein Probeglas mit Urin einstecken und abwarten, bis die Saugeinlage 12 genügend Flüssigkeit angesaugt hat. Dieser Zustand wird optisch sichtbar gemacht, indem sich eine in der oberen Muffe befindliche Zellulosescheibe 41, welche in innigem Kontakt zur oberen Endfläche der Saugeinlage gelagert ist, nach oben entfaltet (vgl. Pos. 41 in Fig. 1).

Die Zellulosescheibe 41 wird über die Saugeinlage 12 mit der gefilterten Körperflüssigkeit getränkt und zeigt das Endfassungs vermögen des Systems an. Der Quellvorgang der Zellulosescheibe 41 wird dadurch sichtbar gemacht, daß die Wandungen der Muffe 26 transparent ausgebildet sind. Die Zellulose kann mit einer gelben oder roten Signalfarbe gekennzeichnet sein.

Statt der als Indikator dienenden Zellulosescheibe 41 kann auch eine aus einem wasserlöslichen Farbstoff bestehende Farbschicht eingefügt werden. Vorzugsweise wird dabei das Ende der Saugeinlage mit einem Farbstoff imprägniert. Der feucht applizierbare und durch einen Trocknungsprozeß an die Saugeinlage gebundene Farbstoff wird nach oben hin mit einer aus Vliespapier gestanzten Scheibe abdeckt. Wenn die Untersuchungsflüssigkeit die Saugeinlage bis zum oberen Pol durchdrungen hat, löst sich der Farbstoff auf und durchdringt die Vliespapierscheibe, wobei sich diese anfärbt. Durch die transparente oder perforierte Oberfläche der Muffe 26 ist die Verfärbung sichtbar.

Damit wird auch angezeigt, daß sich eine hinreichende Ansammlung von Zellmaterial auf der Oberfläche 17 des Zellträgerelementes 10 befindet. Dieses Zellmaterial kann auf einen Objektträger übertragen werden. Bei Bedarf kann dem Sammelvorgang noch ein Fixieren des Zellmaterials mit den üblichen Fixiermitteln, z.B. mit Alkohol, folgen, und zwar vor dem Abstreichen. Die ist dann ist gut möglich, wenn die Körperflüssigkeit z.B. von der zu untersuchenden Person in einer Klinik erhalten wird, und auch in dem Kliniklabor alsbald das weitere Untersuchungsverfahren am Zellmaterial durchgeführt werden kann.

Häufig erfolgt jedoch die Gewinnung der Zellen aus der Körperflüssigkeit in einem zeitlichen und/oder räumlichen Abstand zur Untersuchung. Nicht selten will man auch noch die Möglichkeit zu einer zweiten Proben-Untersuchung haben. Dementsprechend ist die Vorrichtung 1 so ausgebildet, daß sie bedarfsweise einen Verschlußbehälter 3 für das untere Ende 5 des Filterbehälters 2 aufweist (vgl. insbesondere Fig. 1 bis 3). Dabei sind der Verschlußbehälter 3 und der Filterbehälter 2 mit ihren Abmessun gen aufeinander abgestimmt und es ist zwischen ihnen die bereits erwähnte Flüssigkeitsdichtung 13 vorgesehen. Diese Flüssigkeitsdichtung ist dadurch gebildet, daß wenigstens beim unteren Ende des Filterbehälters 2 eine ringförmige Dichtlippe 13 vorgesehen ist.

Wie gut aus Fig. 2 erkennbar ist, ist am oberen Ende 19 des Verschlußbehälters 3 ein Abstreifring 20 vorgesehen, der vorzugsweise aus saugfähigem Werkstoff besteht. Dazu hat der Verschlußbehälter 3 an seinem oberen Ende 19 einen nach außen aufgewölbten, im Querschnitt nahezu U-förmigen Haltewulst 21, die zum Inneren des Verschlußbehälters 3 offen ist. In die dadurch gebildete ringförmige Höhlung ist der Abstreifring 20 eingelegt, der aus saugfähigem Material besteht und, wenn der Filterbehälter 2 in den Verschlußbehälter 3 eingesteckt ist (Fig. 1), die Außenwand des Filterbehälters 2 berührt. Dies hat den Vorteil, daß beim Hereinstecken bzw. beim Herausziehen des Filterbehälters 2 aus dem Vorschlußbehälter 3 die Außenwand des Filterbehälters 2 selbsttätig gereinigt wird und eine Bedienungsperson weitgehend von einer Berührung mit der Flüssigkeit geschützt und somit die Infektionsgefahr erheblich vermindert wird. Dies wird auch noch durch den Griffabschnitt 15 bzw. der zugehörigen Griff-Riffelung des Griffabschnittes 15 begünstigt.

In besonders vorteilhafter Weise wird die Kontamination der Hände des Anwenders dadurch verhindert, daß der Filterbehälter 2 und der Verschlußbehälter 3 geringfügig anders ausgebildet sind.

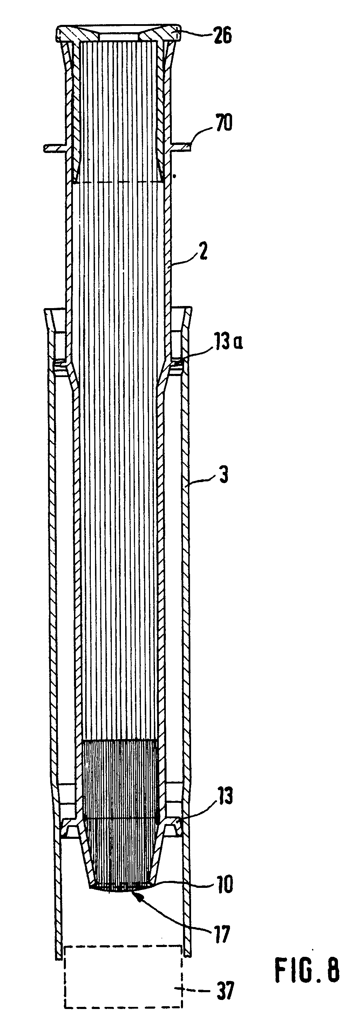

Aus Fig. 8 ist gut erkennbar, daß der Filterbehälter 2 nahe seinem unteren Ende eine umlaufende Dichtlippe 13 aufweist. Eine weitere Dichtlippe 13a ist an der oberen Hälfte des Filterbehälters 2 angebracht. Außerdem ist am Behälter 2 unterhalb der Verschlußkappenzone ein umlaufender Anschlagring 70 vorgesehen. Der Filterbehälter 2 ist von der Mitte aus und zum unteren Ende hin verjüngt. Der Verschlußbehälter 3 ist am unteren Ende ebenfalls verjüngt, so daß sich die Dichtlippe 13 bei zusammengeschobenem Zustand beider Teile in dieser Zone abdichtend einpaßt. Die obere Öffnung des Verschlußbehälters 3 erlaubt ein einfaches Einschieben des Filterbehälters in den Tubus. Der beim Saugprozeß kontaminierte Teil des Filterbehälters 2 verschwindet dabei im Verschlußbehälter 3, wobei die verunreinigte Zone zwischen den Dichtlippen 13 und 13a völlig eingedichtet wird. Das Gesamtsystem kann nun ohne Gefahr für den Anwender gehandhabt und versendet werden, vorausgesetzt, daß die Verschlußkappen 26 und 37 den Verschlußbehälter 3 auch verschließen.

Für die Übertragung von Zellen von der Oberfläche 17 auf einen Objektträger wird die Verschlußkappe 37 vom Verschlußbehälter 3 abgezogen. Der Filterbehälter 2 wird bis zum Anschlagring 70 in den Tubus eingeschoben, wobei sich das Zellträgerelement 10 so weit aus dem Verschlußbehälter 3 herausschiebt, daß sich das Zellmaterial von der Oberfläche 17 problemlos auf den Objektträger (nicht gezeigt) übertragen läßt.

Aus Fig. 1 ist auch erkennbar, daß der Filterbehälter 2 an seinem oberen Ende 4 einen gelochten Deckel 24 hat. Die entsprechende Lochung 25 ist als Einsteck- und Entnahmeöffnung für die Saugeinlage 12 ausgebildet. Sowohl der Filterbehälter 2 als auch der diesen oben begrenzende Deckel 24 müssen eine obere Öffnung 4 bzw. die vorerwähnte Lochung 25 haben, damit die in der Saugeinlage 12 erwünschte Kapillarwirkung zum Ansaugen der Flüssigkeit funktionieren kann. Die Saugeinlage 12 des Filterbehälters 2 ist stabförmig und als axiales Widerlager für die vor ihr liegenden Teile 10 und 11 ausgebildet. Ferner ist die Saugeinlage 12 an ihrem oberen Ende von einer Muffe 26 klemmend eingefaßt, die selbst eine Öffnung nach außen hat, die durch ein Loch 27 an der oberen Stirnseite der Muffe 26 realisiert ist. In dieser Muffe 26 ist über der Saugeinlage 12 und in innigem Kontakt zu dieser eine Zellulosescheibe 41 angeordnet. Diese Zellulosescheibe kann sich nach Feuchtigkeitsaufnahme in der Muffe 26 bis zur Belüftungsöffnung ausdehnen.

Der Vorrichtung 1, wie sie insbesondere gut aus Fig. 1 erkennbar ist, ist ein Verschlußstab 30 zugeordnet (vgl. Fig. 5). Dieser ist in seinen Abmessungen auf die Entnahmeöffnung 25 für die Saugeinlage 12 bzw. auf die dort befindliche Muffe 26 abgestimmt. Nach der Entnahme der Saugeinlage 12 aus dem Filterbehälter 2 kann man den Verschlußstab 30 in die Entnahmeöffnung 25 hineinstecken. Dabei ist der Verschlußstab 30 mit einer Kappe 31 so ausgerüstet, daß er am Filterbehälter befestigbar ist. Dies erfolgt im Ausführungsbeispiel dadurch, daß die Kappe 31 auf wenigstens einem Abschnitt an die Lochung 25 des Deckels 24 so abgestimmt ist, daß der Verschlußstab dort klemmend festzulegen ist. Ferner ist der Verschlußstab 30 mit seinem inneren Ende 32 so bemessen, daß dieses innere Ende ein Widerlager für den Filterabschnitt 11 im Filterbehälter 2 bildet (vgl). Fig. 4 und 5). Durch den auf diese Weise ausgebildeten und im Filterbehälter 2 axial festgelegten Verschlußstab 30 erreicht man eine axiale Festlegung der noch nach dem Entfernen der Saugeinlage 12 im Filterbehälter 2 verbleibende Teile, insbesondere der Teile 10 und 11 bzw. des noch zu erläuternden Teiles 11b.

Der Filterabschnitt 11b ist aus sich bei Kontakt mit Flüssigkeit schwammartig entfaltendem Werkstoff hergestellt. Versuche haben gezeigt, daß sich als Werkstoff regenerierte, verpreßte Zellulose besonders gut eignet. Wenn z.B. ein in Fig. 7 strichpunktiert angedeuteter Teil-Filterabschnitt 11b aus regenerierter, verpreßter Zellulose besteht und mit (Körper-)Flüssigkeit in Verbindung kommt, versucht er sich in erheblichem Umfang auszudehnen, beispielsweise bis zur Frontfläche des Filters 11a (Fig. 7). Durch diese axiale Ausdehnungsbewegung wird die Ansaugwirkung verstärkt. Die Ausdehnungsmöglichkeit des sich axial ausdehnenden Teiles 11b des Filterabschnittes 11 ist einerseits begrenzt durch das unten konische Ende des Filterbehälters 2 in Verbindung mit dem dortigen Zellträgerelement 10 und andererseits nach oben durch das erwähnte Widerlager 32, gebildet aus den Teilen 11a und 12.

Der Filterabschnitt 11, ggf. auch sein aus regenerierter, verpreßter Zellulose bestehender Teilabschnitt 11b, ist mikroporig ausgebildet und er weist im wesentlichen Poren von der Porengröße von maximal 5 μm auf. Diese maximale Porengröße begünstigt, daß möglichst wenig Zellmaterial beim Hochsaugen der Flüssigkeit in den Filterbehälter 2 bzw. seine Saugeinlage 12 hineingelangen kann. Die Saugeinlage 12 besteht aus vorzugsweise in Längsrichtung angeordneten und verpreßten Polyester- oder Zellulosefasern. Diese Saugeinlage 12 hat praktisch keine Filterwirkung, aber eine hohe Saugwirkung.

Wie gut aus Fig. 1 und 2 erkennbar, ist der Verschlußbehälter 3 ebenfalls rohrartig und mit einer oberen und einer unteren Öffnung 35 und 36 versehen. Mit der oberen Öffnung 35 kann der Verschlußbehälter 3 auf das untere Ende 5 des Filterbehälters aufgesetzt werden, der dann den Verschluß der oberen Öffnung 35 bildet. Am unteren Ende 36 des Verschlußbehälters 3 ist eine abnehmbare Verschlußkappe 37 vorgesehen. Diese Verschlußkappe 37 ermöglicht u.a., die Oberfläche 17 des Filterbehälters 2 auch dann noch freizulegen, wenn dieser Filterbehälter 2 sich bereits innerhalb des Verschlußbehälters 3 befindet. Man kann z.B. nachträglich Fixiermittel in den Verschlußbehälter 3 und somit auch auf die Oberfläche 17 bringen und anschließend den Verschlußbehälter 3 wiederum mittels der Verschlußkappe 37 abschließen. Man kann auch zusätzliche zu untersuchende Körperflüssigkeit im sich zwischen der Verschlußkappe 37 und dem unteren Ende 5 des Filterbehälters 2 beim Verschlußbehälter 3 bildenden Hohlraum 38 einbringen. Die Größe des Hohlraumes ist in gewissen Grenzen wählbar, da der Filterbehälter 2 teleskopartig im Verschlußbehälter verschiebbar gelagert ist. Der Verschlußbehälter 3 ist ebenfalls vorzugsweise aus Polyethylen hergestellt und rohrartig ausgebildet und er hat beispielsweise eine Länge L 1 von 110 mm. Unter den bereits erwähnten, am oberen Ende 35 des Verschlußbehälters 3 angebrachten Haltewulst 21 befindet sich der Abstreifring 20, der vorzugsweise aus Schaumstoff besteht. Der Haltewulst 21 ist durch Aufweiten des rohr artigen Verschlußbehälter-Körpers und Umbördeln seines oberen Endes nach innen leicht herstellbar (Fig. 2).

Der aus regenerierter, verpreßter Zellulose bestehende Teil 11b des Filterabschnittes 11 entwikkelt - wie bereits erwähnt -beim Zusammentreffen mit Wasser, Körperflüssigkeit od. dgl. ein sehr starkes Quellverhalten und damit auch eine sehr starke Fähigkeit Flüssigkeit aufzunehmen. Als Beispiel wird für das verwendete Material im gepreßten Zustand eine Dichte von 0,58 g/ccm und im Trokkenzustand nach der Quellung eine Dichte von 0,06 g/ccm gemessen. Wenn die gepreßte Zellulose, die als Teil 11b des Filterabschnittes 11 in Fig. 7 angedeutet ist, mit Wasser bzw. Körper-Flüssigkeit in Berührung kommt, entwickelt sich im Preßling eine Gitterstruktur. Die Porigkeit bzw. deren Größe wird von der Ausdehnungsmöglichkeit des Teiles 11b des Filterabschnittes 11 bestimmt. Beim Ausführungsbeispiel gemäß Fig. 1 und 7 ist die Ausdehnungsmöglichkeit des Teiles 11b des Filterabschnittes 11 dadurch begrenzt, daß die verpreßte Zellulose selbst oben gegen den Filterabschnitt 11a anstößt, der durch einen Ringwulst 33 gehalten wird (Fig. 1).

Gemäß dem erfindungsgemäßen Verfahren arbeitet man mit der Vorrichtung 1 folgendermaßen:

Ein Filterbehälter 2, der sich zunächst noch mit seinem unteren Ende 5 in einem Verschlußbehälter 3 und mit diesem zusammen in einer geschlossenen Verpackung befindet, weist das Zellträgerelement 10, den Filterabschnitt 11, die Saugeinlage 12 und den Deckel 24 auf. Im in Fig. 1 dargestellten, zusammengesetzten Zustand von Filterbehälter 2 und Verschlußbehälter 3 schützt letzterer auch noch besonders die Oberfläche 17 sowie den unteren Teil des Filterbehälters 2, der später in die Körper-Flüssigkeit od. dgl. eingetaucht wird. Danach entfernt man den Filterbehälter 2 aus dem Verschlußbehälter 3 und taucht diesen Filterbehälter 2 mit seinem unteren Ende 5 in einen Behälter ein, in dem sich Körperflüssigkeit (z.B. Urin) befindet, aus der Zellen, Partikel od. dgl. Zellmaterial gesammelt und abgetrennt werden sollen. Zu untersuchende Flüssigkeiten können auch anderer Herkunft sein, z.B. aus einer Bauchhöhlenlavage stammen. Beim Filterbehälter 2 wird dann durch Kapillarwirkung die Flüssigkeit durch die Oberfläche 17 hindurchgesaugt und die zu untersuchenden Zellen und Partikel bleiben auf der Oberfläche 17, die von dem Zellträgerelement 10 aus Acetatgewebe gebildet wird, haften. Der ein- oder mehrteilige Filterabschnitt 11 bzw. 11a und 11b sowie die Saugeinlage 12 saugen die Flüssigkeit durch das Acetatgewebe. Wenn sich z.B. ein Teilabschnitt 11b von regenerierter, verpreßter Zellulose im Filterbehälter 2 befindet, beginnt sich dieser in dem ihm begrenzt zur Verfügung stehenden Raum zu entfalten. Der zur Verfügung stehende Raum und der Werkstoff sind so ausgebildet, daß sich nur Poren bilden, deren Größe in aller Regel kleiner als 5µm ist. Die Flüssigkeit steigt sehr schnell im Filterbehälter 2 hoch, was noch durch die Saugwirkung des zunächst verpreßten Zelluloseteiles 11b begünstigt wird. Schließlich wird diese Flüssigkeit, zumindest zum allergrößten Teil, von der Saugeinlage 12 und der darüber angeordneten Zellulosescheibe 41 aufgenommen und darin gespeichert. Dabei ergibt sich auch als besonders vorteilhafter Effekt, daß sich das mit der Flüssigkeit aufsteigende, zu untersuchende Zellmaterial an der äusseren Schicht des Zellträgerelementes 10, also auf der Oberfläche 17 quantitiv und schonend sammelt. Dabei wird unter "quantitiv" verstanden, daß sich vergleichsweise große Mengen von Zellmaterial auf der Oberfläche 17 ansammeln. Später kann von dort auf das zu untersuchende Zellmaterial durch Abdruck auf einen Objektträger oder auch durch Abspülen in eine Flüssigkeit übertragen. Die Oberfläche 17 kann deshalb als Bereitsstellungsfläche aufgefaßt werden.

Vor dem Abstreifen des zu untersuchenden Zellmaterials wird die Saugeinlage 12 zusammen mit der Muffe 26 entfernt. Anstelle dieser Teile 12, 26 wird der Verschlußstab 30 (Fig. 5) in den Filterbehälter 2 eingesetzt. Dieser Verschlußstab 30 bewirkt eine axiale Lagefixierung des Filterabschnittes 11 und stellt somit auch indirekt ein Widerlager für das Zellträgerelement 10 dar. Versuche haben gezeigt, daß einige Minuten nach dem Eintauchen des Filterbehälters 2 in die zu untersuchende Flüssigkeit etwa 6 ml Probeflüssigkeit durch den Filterabschnitt 11 in die Saugeinlage 12 aufgestiegen sind. Die entsprechende Menge von Zellmaterial hat sich dann auf der Oberfläche 17 niedergeschlagen und kann dann direkt auf einen Objektträger übertragen, gefärbt und mikroskopisch untersucht werden.

Wenn das zu untersuchende Zellmaterial nicht direkt nach dem vorbeschriebenen Vorgang auf einen Objektträger übertragen und entsprechend behandelt werden kann, sondern an ein Zytolaber (Diagnoselabor) zur Untersuchung versandt werden muß, kann wie folgt verfahren werden:

Der Filterbehälter 2 wird in den Verschlußbehälter 3 eingeschoben. Dabei streift sich Flüssigkeit, welche die Außenwand des Filterbehälters 2 kontaminiert hat, am Abstreifring 20 des Verschlußbehälters 3 ab. Damit wird einer Beschmutzung der Hände der Bedienungsperson vorgebeugt. Die Saugeinlage 12 wird nun aus dem Filterbehälter herausgezogen und verworfen. Soll das an der Bereitstellungsfläche 17 haftende Zellmaterial für den Versand konserviert werden, kann die Verschlußkappe 37 des Verschlußbehälters 3 entfernt und eine geeignete Fixierlösung in den Verschlußbehälter 3 eingebracht, ggf. auch speziell die Oberfläche 17 mit einer geeigneten Fixierlösung besprüht werden. Das zu untersuchende Zellmaterial wird also von dem fixierenden Medium benetzt und somit wird verhindert, daß während des Versandes der Vorrichtung 1 eine zellzerstörende Lyse abläuft. Ferner wird in den Filterbehälter ein Verschlußstab 30 eingeführt. Ein solcher Verschlußstab ist an seiner inneren Stirnseite 40 geschlossen und besteht vorzugsweise aus Polystyrol. Der Verschlußstab 30 verhindert, daß das Filtersegment 11 oder ein Teilsegment 11a oder 11b sich verschieben; außerdem verhindert der Verschlußstab 30, daß Flüssigkeit oder andere unerwünschte Bestandteile aus dem Filterabschnitt 11 od. dgl. 11a, 11b austreten oder über diesen Filterabschnitt 11 bzw. 11a und 11b eintreten können. Die Vorrichtung 1 kann dann gut versandt werden.

Wenn es die Aufgabe der Zellgewinnung erforderlich macht, kann der vor der Oberfläche 17 liegende Hohlraum 38 des Verschlußbehälters 3 durch eine entsprechend zurückgezogene Lage des Filterbehälters 2 vergrößert werden, beispielsweise bis zu einem Fassungsvermögen von etwa 10 ml. Dieser Hohlraum 38 kann dann z.B. mit einer zusätzlichen Probeflüssigkeit desselben Patienten gefüllt werden. Die Vorrichtung 1 wird dann mit dem Filterabschnitt 11 nach oben gerichtet aufgestellt und man kann wie folgt verfahren: Der Verschlußstab 30 wird gegen eine neue Saugeinlage 12 vertauscht und die vorerwähnte, zusätzlich zugegebenen Probenflüssigkeit wird von oben nach unten vom Filter angesaugt und die in dieser zusätzlichen Probeflüssigkeit befindlichenen Zellen können, z.B. zusätzlich zu den bereits vorhandenen, auf der Oberfläche 17 abgeschieden werden. Auf diese Weise kann man die für die Untersuchung zur Verfügung gestellte Menge an Zellmaterial vergrößern. Danach erfolgt die Übertragung des Zellmaterials in der üblichen Weise auf den Objektträger.

In Fig. 5 erkennt man am unteren Ende des Verschlußstabes 30 eine im begrenzten Umfang saugfähige Einlage 60. Der Verschlußstab 30 ist im wesentlichen rohrförmig ausgebildet, wobei der Rohrabschnitt mit 30' bezeichnet ist. Nahe seinem inneren Ende hat der Verschlußstab 30 einen ringförmigen, radial nach innen vorstehenden Haltewulst 61, welcher die Einlage 60 in Achsrichtung festlegt. Dementsprechend kann auch die freie, als Widerlager ausgebildete Stirnseite 40 der saugfähigen Einlage 50 wiederum ein Widerlager für die Teile 11 bzw. 11a und 11b (vgl. Fig. 5, 1 und 6 und 7) bilden.

Es kann erwünscht sein, daß noch nach dem Einschieben des Verschlußstabes 30 in den Filterbehälter 2 etwas Flüssigkeit aus dem Filterabschnitt 11 bzw. den entsprechenden Filterabschnitt-Teilen 11a und 11b entfernt werden soll. In diesem Fall ist die Ausführung des Verschlußstabes 30 mit der begrenzt saugfähigen Einlage 60 besonders vorteilhaft.

In einem anderen Fall, bei dem die vorerwähnten Saugvorgänge nicht erforderlich bzw. nicht erwünscht sind, kann das Verschlußrohr 30 unten eine nicht saugfähige Stirnplatte als Abschluß haben.

In Fig. 6 ist unter Weglassung des Filterbehälters 2 selbst dessen Inhalt in auseinandergezogener Darstellung gezeigt. Man erkennt, von oben nach unten in Fig. 6 gesehen, die Saugeinlage 12, einen Filterabschnitt 11 und, mit Abstand davon, ein Zellträgerelement 10 aus dichtem Acetatgewebe.

In Fig. 7 ist der untere Teil des Inhalts des Filterbehälters 2 in etwas abgewandelter Ausführung, ebenfalls in auseinandergezogener Darstellung, wiedergegeben. Man erkennt dort einen verkürzten Filterabschnitt 11a sowie den Filterabschnitt 11b, der aus einem Werkstoff hergestellt ist, der sich bei Kontakt mit Flüssigkeit schwammartig entfalten kann, wie regenerierte, verpreßte Zellulose. Bei der Ausführung nach Fig. 7 sind dann am inneren Ende des Filterbehälters 2 entweder die Platzverhältnisse so gewählt, daß sich der Filterabschnitt 11b auch genügend in einem vorgegebenen Maße volumenmäßig vergrössern kann. Dies kann z.B. durch einen entsprechenden Freiraum oder durch eine gewisse Kompressibilität beim Filterabschnitt 11a erreicht werden.

Die Flüssigkeits-Aufnahmekapazität des Inhaltes des Filterbehälters und ggf. der Scheibe 41 der Hülse 26 sind bei einer bevorzugten Ausbildung der Vorrichtung 1 so auf die zu untersuchende Flüssigkeit abgestimmt, daß dann, wenn die Saugkapazität der Teile im Filterbehälter 2 und ggf. der Scheibe 41 in der Muffe 26 erschöpft ist, auch genügend zu untersuchendes Zellmaterial für die Untersuchung sich auf dem Zellträgerelement 10 bzw. seiner Oberfläche 17 gesammelt hat. Die vorerwähnte Saugkapazität der im Filterbehälter 2 befindlichen Teile wird insbesondere durch die dortige Saugeinlage 12, dem Filterabschnitt 11 bzw. die Teilabschnitte 11a und 11b des Filterabschnittes und ggf. durch die Saugkapazität der in der Muffe 26 befindlichen Zellulosescheibe 41 vorgegeben.

Alle vorbeschriebenen und/oder in den Ansprüchen aufgeführten Merkmale können einzeln oder in beliebiger Kombination miteinander erfindungswesentlich sein.

## Ansprüche

1. Verfahren zum Sammeln und Abtrennen von Partikeln aus einer Flüssigkeit für die medizinische Diagnose,
dadurch **gekennzeichnet,**
daß die Flüssigkeit mittels eines saugfähigen Materials durch eine Oberfläche gesaugt wird, an der die Partikel haften bleiben.

2. Verfahren nach Anspruch 1
dadurch **gekennzeichnet,**
daß Flüssigkeit mittels Kapillarwirkung durch die Oberfläche gesaugt wird.

3. Vorrichtung zum Sammeln und Abtrennen von Partikeln aus einer Flüssigkeit für die medizinische Diagnose, gekennzeichnet durch
- einen ersten Behälter (2), in dem eine die Flüssigkeit ansaugende Saugeinlage (12) angeordnet ist, und
- eine im Saugweg der Flüssigkeit angeordnete Oberfläche (17), die für die Flüssigkeit, nicht aber für die Partikel, durchlässig ist.

4. Vorrichtung nach Anspruch 3,
dadurch **gekennzeichnet,**
daß die Saugeinlage (12) die Flüssigkeit im wesentlichen durch Kapillarwirkung ansaugt.

5. Vorrichtung nach einem der Ansprüche 3 oder 4,
dadurch **gekennzeichnet,**
daß die Oberfläche (17) auf einer von der Saugeinlage gesonderten Schicht, insbesondere einem Kunststoff-, Seiden- und/oder Acetatgewebe, ausgebildet ist.

6. Vorrichtung nach einem der Ansprüche 3 bis 5,
dadurch **gekennzeichnet,**
daß der erste Behälter (2) in einen zweiten Behälter (3) abdichtend einschiebbar ist.

7. Vorrichtung nach einem der Ansprüche 3 bis 6,
dadurch **gekennzeichnet,**
daß im ersten Behälter (2) eine Einrichtung (50) vorgesehen ist, die anzeigt, ob Flüssigkeit in die Saugeinlage (12) gelangt ist.

8. Vorrichtung nach Anspruch 6,
dadurch **gekennzeichnet,**
daß der zweite Behälter (3) mit einer Einrichtung (20, 21) zum Aufsaugen von überschüssiger Flüssigkeit versehen ist.

9. Vorrichtung zum Sammeln und Abtrennen von Partikeln aus einer Flüssigkeit für die medizinische Diagnose,
**gekennzeichnet** durch
- einen Behälter, der in der Lage ist, selbsttätig sein Volumen zu vergrößern, um die Flüssigkeit anzusaugen, und
- eine im Saugweg der Flüssigkeit angeordnete Oberfläche (17), die für die Flüssigkeit, nicht aber für die Partikel, durchlässig ist.

10. Vorrichtung nach Anspruch 9,
dadurch **gekennzeichnet,**
daß der Behälter sein Volumen unter Wirkung einer Feder oder aufgrund seiner Eigenelastizität vergrößert.

FIG. 1

FIG. 2

FIG. 3

FIG. 5

FIG. 4a

FIG. 4

FIG. 7

FIG. 6

FIG. 8